# EUROPEAN PATENT APPLICATION

(11) **EP 2 772 758 A1**
(43) Date of publication of application: **03.09.2014**
(21) Application number: 13156865.1
(22) Date of filing: 27.02.2013
(51) Int. Cl.: G01N 33/574

(54) **EpCAM tumour diagnosis**

(71) Applicant: Oncotyrol Center for Personalized Cancer Medicine GmbH, 6020 Innsbruck (AT)
(72) Inventor: Spizzo, Gilbert, 39012 Meran (IT); Fong, Dominic, 39028 Kortsch/Schlanders (IT)
(74) Representative: Sonn & Partner Patentanwälte

(57) **Abstract**

The invention discloses a method for in vitro diagnosing a pancreas or colon cancer tissue specimen wherein the specimen is analysed for the presence or absence of the ectodomain of EpCAM (EpEX) as well as for the presence or absence of the intracellular domain of EpCAM (EpICD).

SEQUENCE LISTING

<110>  ONCOTYROL - Center for Personalized Cancer Medicine GmbH

<120>  EpCAM TUMOUR DIAGNOSIS

<130>  R 61495

<160>  1

<170>  PatentIn version 3.5

<210>  1
<211>  11
<212>  PRT
<213>  artificial

<220>
<223>  EpCAM peptide

<400>  1

Cys Met Gly Glu Met His Arg Glu Leu Asn Ala
1               5                   10

## Description

The present invention relates to the field of tumour prognosis.

Characterised by its aggressive tumour biology most pancreatic cancer patients already have locally advanced or metastatic disease at the time of diagnosis. While surgical resection offers the only potentially curative treatment, the majority of patients experience recurrence within a short period even after complete (R0) resection. Current Gemcitabine-based chemotherapy provides only marginal survival benefit, and even the addition of novel, molecularly targeted therapies do not prolong patient survival significantly. Moreover, in advanced and metastatic pancreatic cancer more recent trials have included the control of disease-related symptoms defined by the clinical benefit response rate (CBR) as an endpoint for evaluating therapeutic success in this disease. Therefore, definition of reliable prognostic and predictive markers is fundamental to allow the selection of patients who are most likely benefit from novel therapeutic approaches.

Tumour antigen specific monoclonal antibodies (mAb) have been successfully implemented into standard treatment regimens for patients with different malignant diseases. However, clinical efficacy of these cancer specific therapies is only seen in a subset of patients. Moreover, only little is known about the mechanisms underlying the clinical response of patients to mAb-based immunotherapy.

The epithelial cell adhesion molecule/antigen EpCAM (also known as GA733-2, TACSTD1, ESA, KS1/4) is a 40 kDa protein that is frequently expressed in normal epithelia and that has been described to be widely overexpressed in carcinomas of various origin. As a consequence, the presence of high amounts of membranous EpCAM in multiple tumours, have rendered EpCAM an ideal target for antibody-based therapeutics. For this reason, EpCAM was described being one of the first antigens to be targeted by monoclonal antibodies in cancer patients. While the murine monoclonal anti-EpCAM antibody Edrecolomab (17-1A, Panorex^{®}) showed anti-tumoural activity in gastrointestinal cancer patients, clinical trials are currently evaluating different immunotherapeutic approaches against this common tumour antigen. Only recently, the trifunctional EpCAM/CD3-specific antibody Catumaxomab could show meaningful clinical activity by the addition of co-adjuvant mechanisms and has been approved for the treatment of malignant ascites in cancer patients with EpCAM-positive tumours. In a phase II/III study, quality of life was improved as symptoms of ascites were significantly decreased compared with the control group.

However, despite its broad distribution in human malignancies, EpCAM-specific therapeutic antibodies still have not hold promise and results from recent clinical trials have been disappointing. Therefore, identification of the mechanisms underlying the therapeutic efficacy of EpCAM targeted therapies is crucial to optimize the selection of patients most likely to benefit from their use.

The object of the present invention is to provide improved means for diagnosing cancer patients and provide significant prognostic and/or predictive markers which allow a personalised optimization of treatment regimen.

Therefore, the present invention provides a method for in vitro diagnosing a pancreas or colon cancer tissue specimen wherein the specimen is analysed for the presence or absence of the ectodomain of EpCAM (EpEX) as well as for the presence or absence of the intracellular domain of EpCAM (EpICD).

Therapeutic antibodies that target EpCAM usually bind the ectodomain of the antigen termed EpEX but do not recognize the intracellular domain EpICD. For this reason the present invention applies EpICD specific antibodies which assess membranous EpICD expression by immunohistochemistry. Such expression analyses can be compared with the staining patterns obtained by conventional EpEX antibodies. The present invention specifically investigated in the membranous presence of full-length EpCAM or truncated variants in pancreatic cancer and defined the prognostic impact thereof.

In the course of the present invention it could be shown that expression of membranous EpEX/EpICD expression is varying significantly in different tumour tissue. In fact, there are tumours in which presence of membranous EpICD is lost, whereas in other tumours, such loss is not observed. This loss of presence of membranous EpICD has biological and clinical relevance, because patients who show such loss have to face a worse development (disease course) than those who show EpICD presence. In fact, the data gathered in the course of the present invention show that median overall survival is significantly different for the two groups of patients. According to the present invention, patients who show a loss of presence of membranous EpICD have a worse prognosis than patients who have membranous EpICD in the cancer tissue specimen. This allows a targeted more aggressive treatment of the patients with a worse prognosis, whereas patients with a good prognosis can be relieved from such an aggressive treatment. This has been shown according to the present invention for tissue cancer specimen from patients with pancreas carcinoma and for patients with colon carcinoma. From the medical point of view, it is clear that patients with membranous EpEX+/EpICD- phenotype metastatic pancreatic cancer showing reduced survival should not be treated with the antibody Catumaxomab or antibodies with similar EpCAM specificity, since these patients are not supposed to benefit from such a treatment. On the other side, pancreatic cancer patients with EpEX+/EpICD+ phenotype are more appropriated for such a treatment and should therefore be treated with Catumaxomab (or antibodies recognising the same epitope as Catumaxomab (Removab^{®}; a trispecific antibody; mouse IgG2a/rat IgG2b hybrid), such as Proxinium^{®}, Vivendium^{®} (VB4-845; immunotoxin; single-chain antibody pseudomonas exotoxin fusion), IGN-101 (edrecolomab; a vaccine for induction of antiidiotypic antibody response), Adecatumumab (MT201; fully human IgG1 mAb), ING-1 (human engineered IgG1 mAb), EMD 273 066 (huKS-IL2; fusion of humanized mAb KS1/4 with human IL-2); see: Baeuerle et al., BJC 96 (2007): 417-423). Moreover, pancreatic cancer patients with EpEX+/EpICD- phenotype are candidates for - compared to the standard treatments (e.g. Seeber/Schutte "Therapiekonzepte Onkologie" 5th edition (2007): 713ff., 747ff.) - more aggressive chemotherapy regimens (e.g. GemOx (Louvet et al., JCO 20 (2002): 1512-1518), GemCap (Cunningham et al., JCO 27 (2009): 5513-5518) or Folfirinox (Conroy et al., New England Journal of Medicine 364 (2011): 1817-1825)) and potentially for inhibitors of EpCAM cleavage (Maetzel el al., Nat. Cell Biol. 11 (2009): 162-171) such as TACE inhibitors (for review see: Das Gupta et al., Bioorg. Med. Chem. 17 (2009): 444-459).

For patients with colorectal cancer, many clinical studies have been performed with the anti-EpCAM antibody Edrecolomab. Most of these studies have not shown improved survival of these patients (Niedzwiecki et al., J. Clin. Oncol. 29 (2011): 3146-3152). According to the teachings of the present invention, it is evident that the efficacy of such a treatment is limited to patients with EpEX+/EpICD+ phenotype and that the studies might have failed upon bad selection of patients.

Preferred cancer tissue specimens according to the present invention are tissue sections and cytocentrifugates from pleura and ascites fluids. Preferred tissue section specimens are paraffin-embedded tissue sections and fresh frozen sections.

Regulated intramembrane proteolysis activates EpCAM as a mitogenic signal transducer in vitro and in vivo. This involves shedding of its ectodomain EpEX and nuclear translocation of its intracellular domain EpICD.

Determination of EpCAM in cancer tissue specimen according to the present invention can, in principle, be established by various methods including immunohistochemistry (Spizzo et al., 2004; Slanchev et al., PLoS Genetics 5 (2009), e1000563; WO 2011/032296 A1, PCT/EP2012/062397), ELISA (Abe et al., J. Immunol. Meth. 270 (2002), 227-233; Petsch et al., mAbs 3 (2011), 31-37); Schmetzer et al, Immunol. Lett. 143 (2012), 184-192), immunofluorescence, immunoprecipitation (Maetzel et al., Nat. Cell Biol. 11 (2009), 162-171), flow cytometry, etc..

The method according to the present invention has turned out to be highly relevant for correlating the prognosis and aggressiveness of treatment of patients suffering from colon carcinoma and pancreas cancer. The present invention therefore specifically provides a method for in vitro diagnosing a colon carcinoma (CC) tissue specimen or a pancreas cancer (PC) tissue specimen wherein the specimen is analysed for the presence or absence of the ectodomain of EpCAM (EpEX) as well as for the presence or absence of the intracellular domain of EpCAM (EpICD).

As already mentioned, the presence of EpEX and the absence of EpICD in the specimen is indicative of a shortened disease-free survival of a patient with CC or PC from which the specimen had been obtained, preferably for patients, with exocrine pancreas cancer, especially patients with pancreatic ductal adenocarcinomas. Endocrine pancreas cancers or other special forms of pancreas cancer have other progression mechanisms and are less suited.

Preferably, the presence or absence of EpEX or the presence or absence of EpICD is analysed by use of an antibody binding specifically to EpEX and/or EpICD, respectively. Specificity of the antibodies to EpEX and EpICD have therefore to be confirmed carefully (e.g. the antibodies M2-5 and M4-10 of Abe et al. (2002) are not able to bind to EpICD).

Antibodies for use in the present invention include synthetic antibodies, monoclonal antibodies, polyclonal antibodies, recombinant antibodies, antibody fragments (such as Fab, Fab', F(ab')₂), dAb (domain antibody), antibody heavy chains, intrabodies, humanized antibodies, human antibodies, antibody light chains, single chain Fvs (scFv) (e.g., including monospecific, bispecific etc), anti-idiotypic (ant-Id) antibodies, proteins comprising an antibody portion, chimeric antibodies (for example, antibodies which contain the binding specificity of murine antibodies, but in which the remaining portions are of human origin), derivatives, such as enzyme conjugates or labelled derivatives, diabodies, linear antibodies, disulfide-linked Fvs (sdFv), multispecific antibodies (e.g., bispecific antibodies), epitope-binding fragments of any of the above, and any other modified configuration of an immunoglobulin molecule that comprises an antigen recognition site of the required specificity. An antibody includes an antibody of any type (e.g. IgA, IgD, IgE, IgG, IgM and IgY), any class (e.g. IgG1, IgG2, IgG3, IgG4, IgA1 and IgA2), or any subclass (e.g. IgG2a and IgG2b), and the antibody need not be of any particular type, class or subclass. In certain embodiments of the invention the antibodies are IgG antibodies or a class or subclass thereof. An antibody may be from any animal origin including birds and mammals (e.g. human, murine, donkey, sheep, rabbit, goat, guinea pig, camel, horse, or chicken).

A "recombinant antibody" includes antibodies that are prepared, expressed, created or isolated by recombinant means, such as antibodies expressed using a recombinant expression vector transfected into a host cell, antibodies isolated from recombinant, combinatorial antibody libraries, antibodies isolated from an animal (e.g. a mouse or cow) that is transgenic and/or transchromosomal for human immunoglobin genes, or antibodies prepared, expressed, created or isolated by any other means that involves slicing of immunoglobulin gene sequences to other DNA sequences.

It is even more preferred to analyse the presence or absence of EpEX and the presence or absence of EpICD by use of antibodies binding specifically to EpEX and EpICD, respectively, and wherein the antibodies are preferably monoclonal antibodies.

A "monoclonal antibody" refers to an antibody obtained from a population of homogenous or substantially homogenous antibodies. Generally each monoclonal antibody recognizes a single epitope on an antigen (i.e. EpEX or EpICD). In aspects of the invention, a monoclonal antibody is an antibody produced by a single hybridoma or other cell, and it specifically binds to only EpEX or EpICD as determined, for example by ELISA or other antigen-binding or competitive binding assay known in the art. The term is not limited to a particular method for making the antibody and for example they may be produced by the hybridoma method or isolated from phage libraries using methods known in the art.

Antibodies including monoclonal and polyclonal antibodies, fragments and chimeras, may be prepared using methods well known to those skilled in the art. Isolated native or recombinant Polypeptide Thyroid Cancer Markers may be utilized to prepare antibodies. Antibodies specific for EpEX or EpICD may also be obtained from scientific or commercial sources. In an embodiment of the invention, antibodies are reactive against EpEX or EpICD if they bind with a Kₐ of greater than or equal to 10⁻⁷ M.

Preferably, the antibodies used for binding specifically to EpEX and/or EpICD are labelled.

According to a preferred embodiment of the present invention, the antibody specifically binding to EpICD and the antibody specifically binding to EpEX are labelled with different labels, preferably with different fluorescence labels.

Preferably, the binding of the antibodies to EpEX and/or EpICD is detected by using secondary antibodies binding to the antibodies binding to EpEX and/or EpICD, said secondary antibodies being preferably labelled, especially with a fluorescence label, a luminescence label, a colourigenic label; a radioactive label, a biotin label, or combinations thereof.

The method according to the present invention is preferably designed as an enzyme linked immunosorbent assay (ELISA), an immunocytochemistry assay, a Western blot assay, a detection of circulating tumour cells or tumour stem cells assay, or a detection of circulating cells or stem cells assay.

According to a preferred embodiment, the method comprises mass spectroscopy. According to another aspect, the present invention relates to a kit for detecting EpEX and EpICD in an epithelial cancer tissue specimen in a method according to the present invention, comprising:
- an epithelial cancer tissue specimen,
- an antibody specifically binding to EpICD; and
- an antibody specifically binding to EpEX.

The kit according to the present invention preferably further comprises detection agents for detecting the antibodies and/or detection means for detection of a binding event between the antibodies to EpICD and/or EpEX. Preferably, the antibodies are monoclonal antibodies.

Preferably, the kit further comprises EpICD and EpEX standards, preferably recombinant EpCAM protein, a recombinant EpICD protein, a recombinant EpEx protein, or combinations thereof.

According to a preferred embodiment, the kit according to the present invention additionally contains positive and negative control tissue specimens.

The invention is further illustrated by way of the following example and the figures, yet without being restricted thereto.

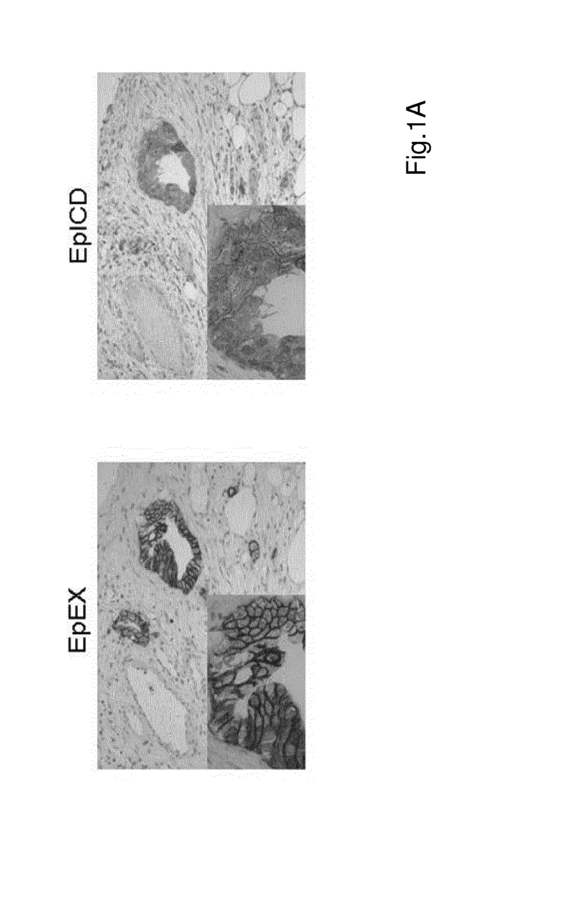

Figure 1A shows representative expression of EpEX and EpICD on whole-mount tissue from a patient with pancreatic cancer. Whole-mount tissue sections from both normal and tumour samples were used to optimize the EpICD staining protocol. The significant loss of EpICD expression on the membrane of tumour cells has to be specifically noted.

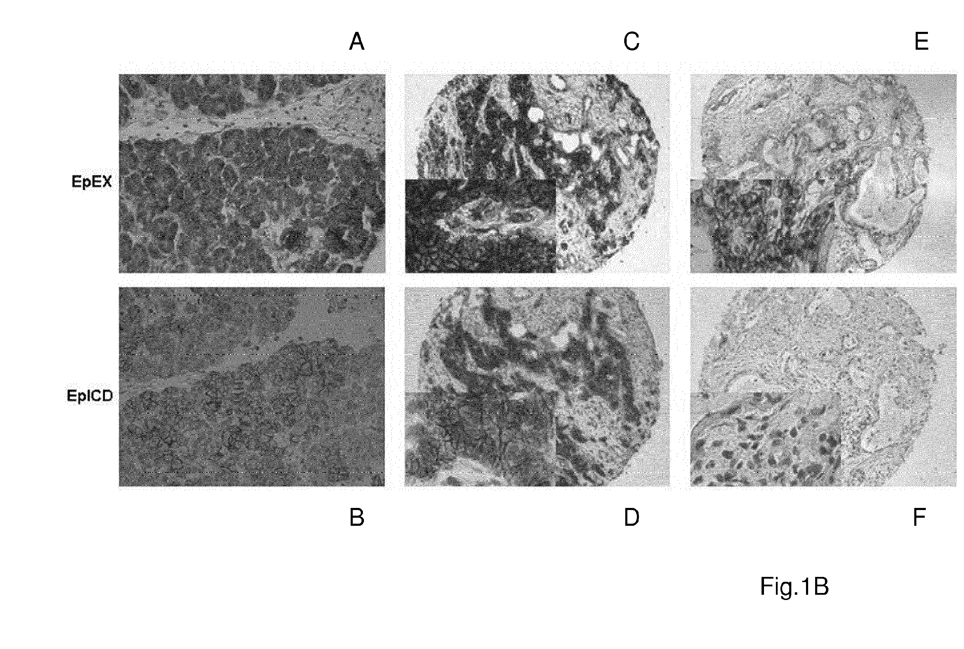

Figure 1B shows the immunohistochemical analysis of EpEX and EpICD in normal and pancreatic cancer tissue; original magnification x 100. (A and B). Normal pancreatic tissue showing moderate to strong membranous EpEX and EpICD expression with an overall concordance of 82% (C and D). Pancreatic ductal adenocarcinoma with concordant EpEX and EpICD expression and loss of membranous EpICD expression (E and F). The cytoplasmic expression of EpICD in tumour cells with loss of membranous EpEX expression follicles (arrow) has to be specifically noted.

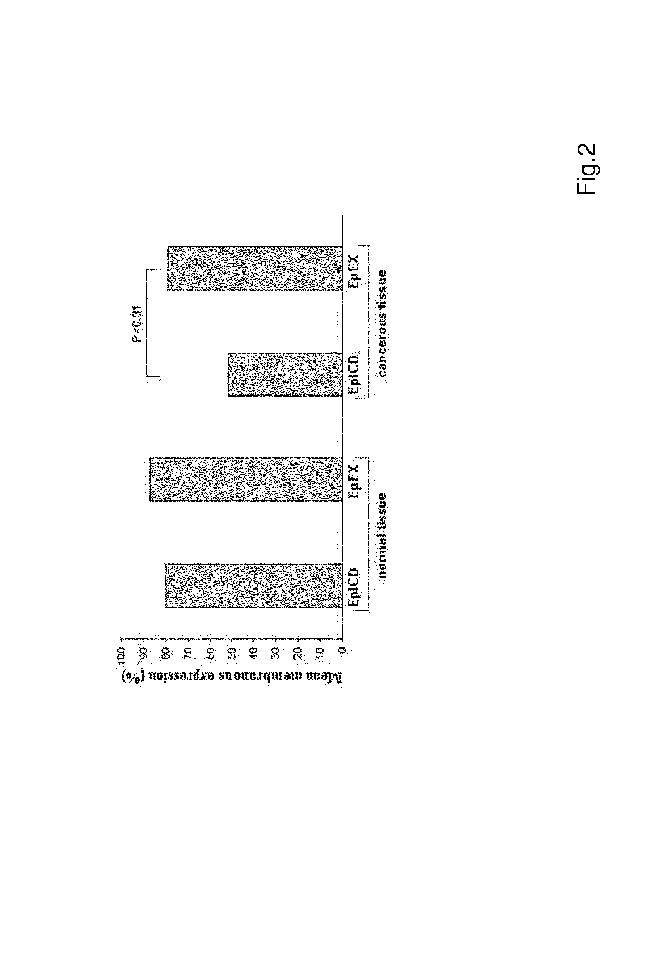

Figure 2 shows mean membranous expression of EpEX and EpICD in normal and pancreatic cancer tissues. Overall concordance between EpEX and EpICD in normal and cancer specimens was 82% and 71%, respectively (κ=0.67 and κ=0.53; p<0.01). A significant loss of membranous EpICD expression in cancer specimens (p<0.01) has to be specifically noted.

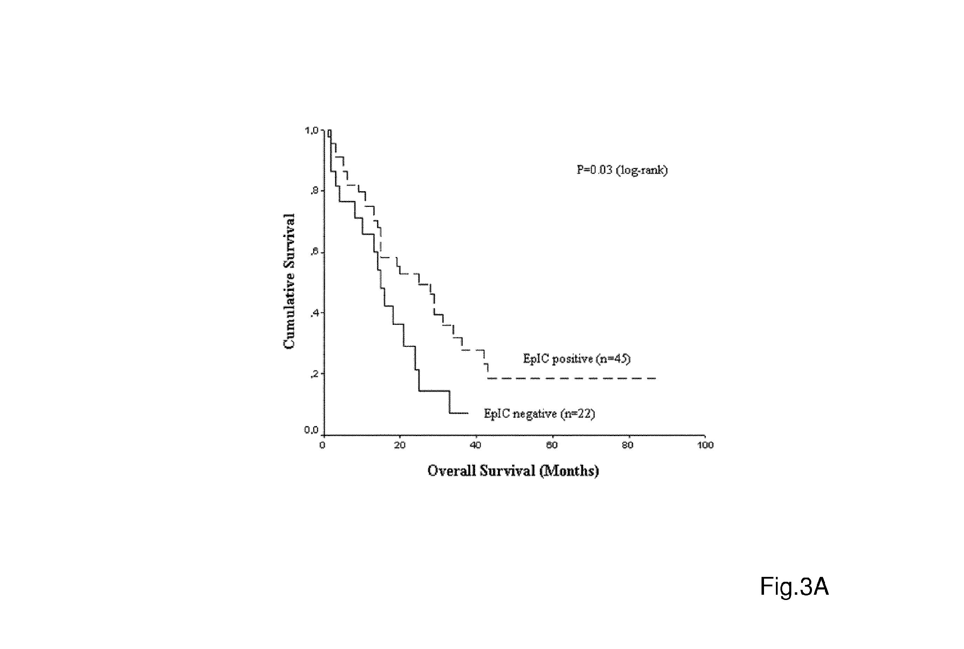

Figure 3A shows prognostic significance of EpICD antigen expression in 67 patients with pancreatic ductal adenocarcinomas regarding overall survival as calculated by Kaplan-Meier analysis. Positive, patients with tumour (n=45) with membranous EpICD expression; Negative, patients with tumour (n=22) with loss of membranous EpICD expression. Patients with positive EpICD expression had significantly better overall survival than did patients with negative EpICD expression as defined by log-rank test (p=0.03).

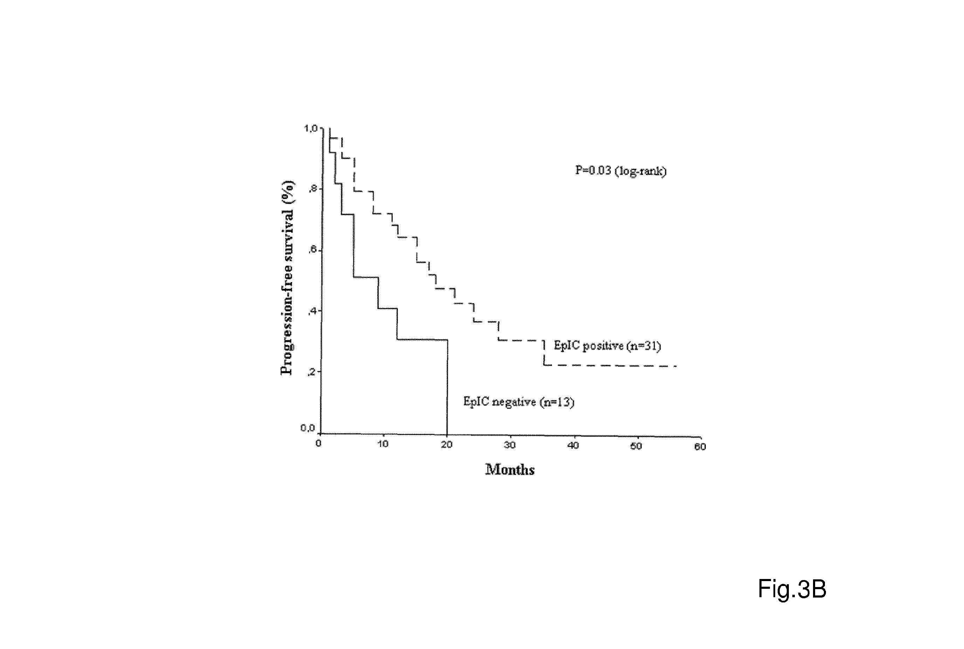

Figure 3B shows prognostic significance of EpICD antigen expression in 44 patients with pancreatic ductal adenocarcinomas regarding progression free survival as calculated by Kaplan-Meier analysis. Positive, patients with tumour (n=31) with membranous EpICD expression; Negative, patients with tumour (n=13) with loss of membranous EpICD expression. Patients with positive EpICD expression had significantly better overall survival than did patients with negative EpICD expression as defined by log-rank test (p=0.03).

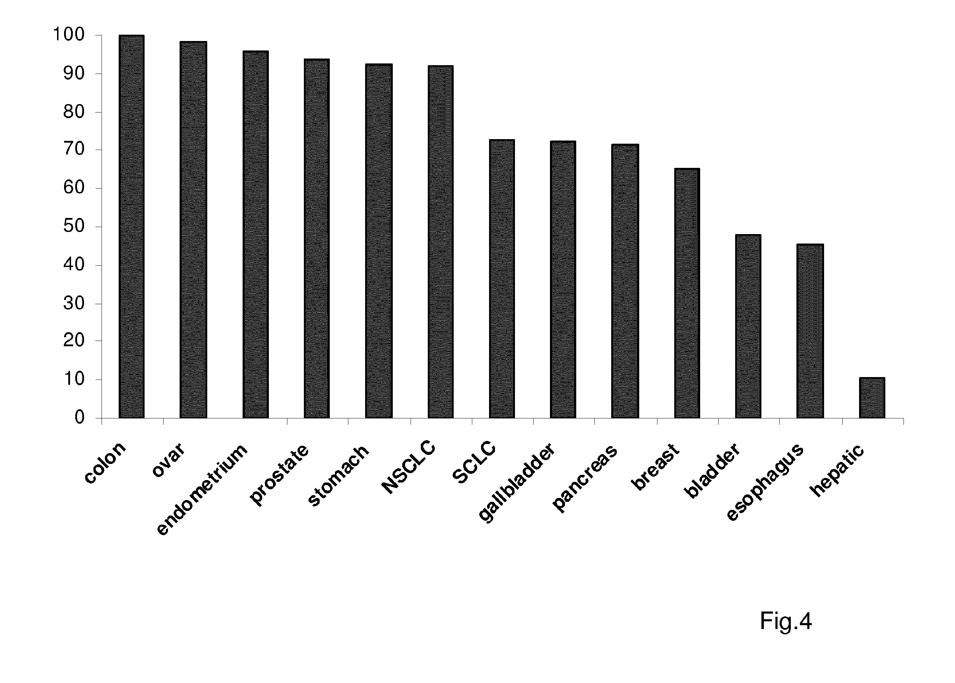

Figure 4 shows % of EpCAM (EpEX⁺) positive tumours; EpCAM⁺ and EpCAM being 100%.

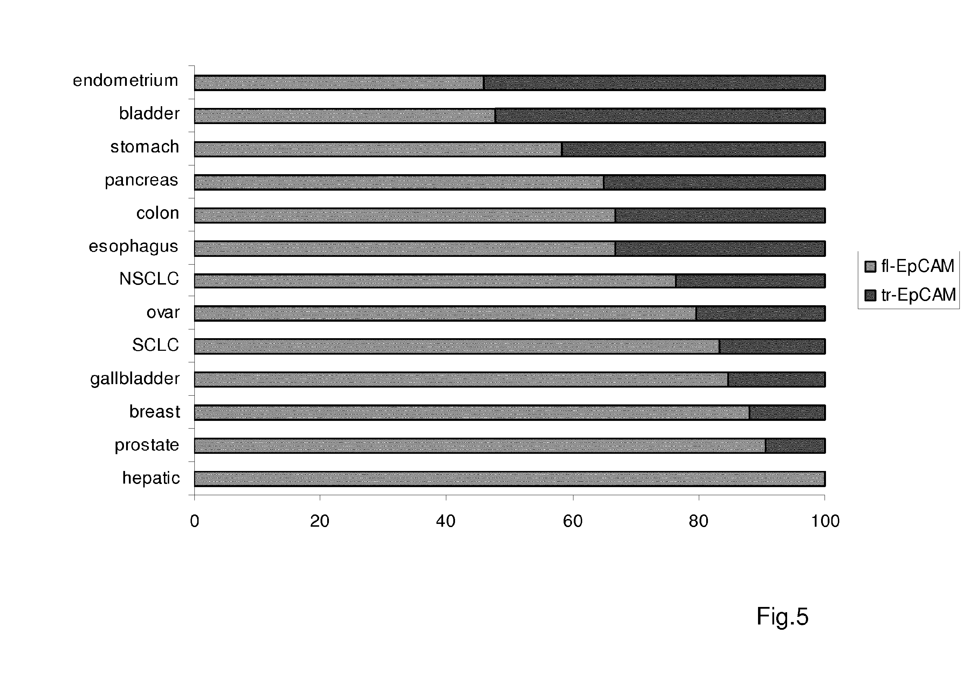

Figure 5 shows % analysis of EpCAM⁺ tumours with respect to the amount of cases with full length EpCAM ("EpCAM^{fl}" (EpEX⁺/EpICD⁺)) and the amount of cases with truncated EpCAM ("EpCAM^{tr}" (EpEX⁺/EpICD⁻)) being 100%.

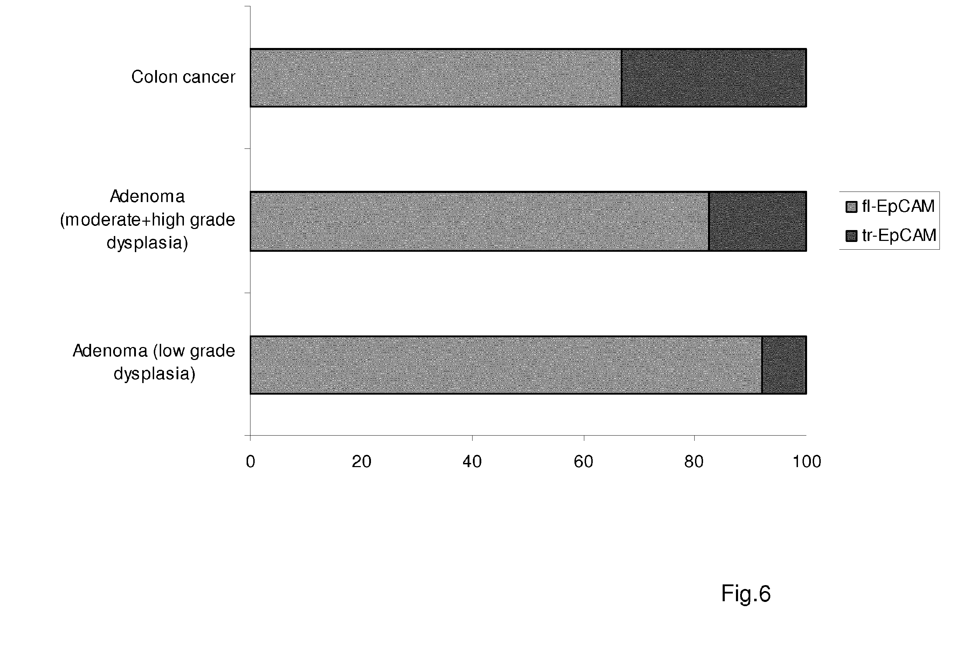

Figure 6 shows loss of EpICD in colon adenoma vs. colon cancer (N=150; p=0.024 (χ²-Test)).

### Example

Loss of membranous EpICD expression is a frequent event and predicts poor survival in patients with EpEx-positive pancreatic cancer

Pancreatic cancer is a fatal neoplasm and current treatment regimens do only marginally prolong patient survival. As a consequence, there is an urgent need for new prognostic biomarkers as well as for therapeutic targets. EpCAM is a widely used immunohistochemical marker for epithelial human malignancies and recently the new EpCAM-specific antibody Catumaxomab has been approved also for the treatment of malignant ascites in pancreatic cancer patients. Antibodies to target EpCAM are usually directed against its ectodomain EpEX but do not detect the intracellular domain EpICD. For this reason a sequence-specific anti-EpICD antibody was developed to compare membranous EpEX vs. EpICD expression by immunohistochemistry.

In the present example, concurrent EpEX and EpICD expression was investigated retrospectively in paraffin-embedded primary tumour tissue samples from a series (n=89) of patients with pancreatic adenocarcinoma on a tissue microarray. EpEX/EpICD expression was correlated with clinicopathological parameters and clinical outcome.

In non-cancerous tissue, a high concordance of membranous EpEX and EpICD expression was observed. However, in tumour tissue 27% of EpEX positive specimens showed loss of membranous EpICD expression. Patients with EpEX⁺/EpICD⁻ phenotype were observed to have a significant shortened disease-free and overall survival independently from other prognostic factors.

Taken together, the present example demonstrates for the first time, that loss of membranous EpICD expression is a frequent event and predicts poor prognosis in patients with pancreatic cancer.

### Materials and Methods

### Patients and Tissue Samples

This study was conducted according to the regulations of the local Ethics Committee and Austrian law. A total number of 89 randomly selected patients with pancreatic ductal adenocarcinoma, from whom formalin-fixed, paraffin-embedded tissue samples were available, diagnosed between 2000 and 2006 at the Department of Pathology, Medical University Innsbruck were included in this retrospective study. Patients with other pancreatic malignancies such as intraductal papillary mucinous adenocarcinoma, acinar cell carcinoma and malignant endocrine tumours were excluded. All tumour specimens were reclassified on hematoxylin and eosin-stained slides and histological type and tumour grade were reassessed by a pathologist using standard diagnostic criteria. Clinical data were obtained by reviewing the charts and contacting the physicians in charge. Tumours were histologically classified according to the WHO classification and staged according to the TNM classification (Schmidt et al., Ann. Oncol. 21 (2010): 275-282). Overall survival was defined as the period of time from initial diagnosis to death or last contact, i.e. date of last follow-up visit. Progression-free survival was defined as the time from surgery with curative intent to appearance of local disease recurrence or evidence of metastatic lesions detected by computed tomography.

### Tissue microarray construction

A representative tumour section paraffin block (donor block) was collected from each case. Viable areas of tumour specimens were marked by one pathologist. At least 3 tumour core biopsies as well as 1 core with normal pancreatic tissue (1mm diameter) were obtained from each specimen using a manual arrayer (Beecher Instruments, Silver Springs, MD, USA). Trephined paraffin tissue cores were consecutively placed in recipient blocks. Each tissue array block contained up to 192 cores, allowing a total of 576 cases to be contained in 3 array blocks. Cores containing tumour tissue in more than 10% of the core area were considered. Sections of 5-µm thickness were cut from each tissue array block and stained with hematoxylin and eosin.

### Generation of a monoclonal Antibody against EpICD

The peptide sequence CMGEMHRELNA-OH (SEQ.ID.No.1) from the C-terminus of the intracellular domain of human EpCAM was selected as target for generating the monoclonal antibody 9-2. BALB/c mice (female, approx. 8 weeks) from Janvier (France) were used to generate hybridomas. For fusion, SP2/0-Ag14 cells (Deutsche Sammlung von Mikroorganismen und Zellkulturen) were used. After immunisation and test of serum, isolated thymocytes were fused with SP2/0 cells. Positive clones were further subcloned. Immunisation, fusion and subcloning was performed by Biogenes GmbH, Berlin. Peptides for immunisation were produced by Biosyntan GmbH, Berlin.

Hybridoma were grown in ISF-1 medium (Biochrom AG, Berlin) and supernatants were harvested and sterile filtered. Antibodies were isolated from the supernatant by protein G chromatography and subsequently concentrated and rebuffered in phosphate buffered saline at pH 7, sterile filtered and aliquots thereof were stored at -35°C. Purity of mAbs was controlled by SDS-PAGE and concentration of mAbs was determined with a ND-1000 spectrophotometer (Peqlab Biotechnologie GmbH, Erlangen).

### Anti-EpICD antibody validation

Antibody specificity was also confirmed on histological tissue sections by pre-absorption with the peptides. The antibody was diluted to its working concentration and incubated with a tenfold excess of relevant peptide overnight at 4°C before application on the tissue. To optimise the EpICD staining protocol, full sections of paraffin-embedded tissue from both normal and tumour samples were stained using a range of primary antibody dilutions, incubation times and antigen retrieval methods and were compared to commercial available anti-EpCAM antibodies.

### Immunohistochemistry

Immunohistochemistry (IHC) of tumour samples with the antibody clone directed against the extracellular domain of EpCAM (EpEx) MOC31 was performed according to manufacturer's instructions. This staining protocol is used routinely in the evaluation of pathologic specimens.

Immunostaining using the EpCAM antibody (clone 9-2) binding to the intracellular domain of EpCAM (EpICD) was performed as follows:
Three µm thick sections from formalin-fixed paraffin-embedded tissue specimens were stained using the automated immunostainer Leica BOND-MAX™ (Leica Microsystems, Wetzlar, Germany). Leica provides specifically for this staining machine all the solutions used, with the exception of the primary antibody clone 9-2 directed against EpICD. Following steps were programmed on the staining machine: dewaxing at 72°C, antigen retrieval with ER2 (epitope retrieval pH9) 20 minutes at 100°C, wash solution, peroxide blocking solution for 5 minutes at room temperature, wash solution, treatment with the primary antibody 9-2 against EpICD at a concentration of 1:5, wash solution, post primary antibody treatment over 8 minutes at room temperature, wash solution, Leica BOND-MAX™ Polymer treatment over 8 minutes at room temperature, wash solution, Mixed DAB treatment over 10 minutes at room temperature, washing with distilled water, counterstaining with HTX over 8 minutes at room temperature, washing with distilled water, dehydration in alcohol and xilene and mounting on glass slides with Entelan.

### Scoring

EpCAM antigen expression was evaluated by two independent observers using light microscopy in a blinded fashion. Discordant cases were reevaluated on a double-headed microscope to achieve a consensus. Antigen expression was defined as the presence of specific staining on surface membranes of tumour cells as described previously (Baeuerle et al., Br. J. Cancer 96 (2007): 417-423). Briefly, membranous expression of EpEx and EpICD was scored as 0=negative, 1=weak, 2=moderate or 3=strong based on staining intensity in at least 10% of tumour cells. The median staining intensity of the tumour cells was calculated for each case and categorized as 0, (no reactivity); 1+ (median reactivity >0-1); 2+, (median reactivity >1-2) and 3+, (median reactivity >2). Cases with median intensity scores ≥1+ were considered positive for EpEX and EpICD expression. Concordance rate of EpEX and EpICD expression was assessed for normal tissue and tumour cells.

### Multi-Tumour Tissue Microarray

For multi-tumour tissue microarray construction, a hematoxylin and eosin-stained slide was made from each block to define representative tumor regions. Tissue cylinders (cores) with a diameter of 0.6 mm were punched from the tumor areas of each block and brought into a recipient paraffin block using a precision instrument (Beecher Instruments, Silver Spring, MD). Five-micrometer sections of these tissue microarray blocks were transferred to an adhesive-coated glass slide system (Instrumedics Inc., Hackensack, NJ). The microarrays contained more than 10 cases from each of most important human tumour entities.

### Statistical Analysis

Statistical analysis was performed using the Statistical Package of Social Sciences (SPSS, version 10.0, Chicago IL). The κ-coefficient was used to assess concordance for EpEX and EpICD status. The statistical significance of the change in membranous EpEX and EpICD expression was analyzed by the Wilcoxon signed-rank test. Correlations between EpICD expression and clinicopathological variables were assessed with the chi-square test. Survival rates were calculated using the Kaplan-Meier method and compared by means of the log-rank test. Follow-up time was censored if the patient was lost during follow-up. Factors with prognostic significance in the univariate models were further evaluated in a multivariate Cox regression model. For all analyses, a P-value of less than 0.05 was considered statistically significant.

### Results

### Patient characteristics

Demographic data and tumour characteristics are summarized in Table 1. At the time of last clinical follow-up (February 2008), 62 (70%) patients out of the total group had died. The overall median survival time for the entire cohort was 14 months (range, 1-88). Most patients (88%) had undergone primary surgical intervention, whereas 11 (12%) patients were considered inoperable. Palliative surgery included palliative bypass or endoscopic bile duct stenting. If indicated, patients received standard Gemcitabine-based chemotherapy according to their clinical stage and performance status.

### Immunohistochemistry

Representative micrographs of tumours with predominant membranous staining of EpEX/EpICD are shown in Figure 1. Different patterns of membranous and cytoplasmic EpEX and EpICD expression occurred across non-neoplastic and pancreatic cancer specimens. Particularly, membranous EpEX expression was observed in 86% of normal pancreatic tissue and in 79% of cancer specimens, respectively. In contrast, membranous EpICD expression was observed in 81% of normal pancreatic tissue and in 52% of cancer specimens, respectively. The overall concordance between EpEX and EpICD expression in normal pancreatic tissue and tumour specimens was 82% and 71%, respectively (κ=0.67 and κ=0.53; p<0.01). However, loss of membranous EpICD expression was observed in 27% of cancer specimens (Figure 2). Interestingly, in the majority of these cases a considerable cytoplasmic expression of EpICD was observed. To further evaluate the significance of loss of EpICD in EpEX positive tumours, a detailed analysis for the subgroup of EpEX positive pancreatic cancer specimens (n=68) was performed. For this reason, EpEX⁻/EpICD⁻ cases were excluded from the analysis.

### Clinicopathological variables and patient survival in EpEX positive tumour specimens

By univariate analyses loss of membranous EpICD expression were compared to age, sex, tumour grading and stage (Table 2). No correlation between these clinical or pathological features and EpICD expression was found. To evaluate a potential correlation of loss of membranous EpICD expression with the survival of pancreatic cancer patients, Kaplan-Meier analysis and the log-rank test for censored survival data was applied. Consistent with previous findings, early tumour stage and a negative resection margin were associated with longer overall survival (Table 1). Of note, loss of membranous EpICD expression was significantly correlated with poor overall survival (p=0.03; Figure 3A) and a shorter progression free interval (p=0.03; Figure 3B). Median overall survival time for patients with tumour presenting with EpICD expression was 25 months compared to 15 months in patients with loss of EpICD expression. Subsequent multivariate analysis identified loss of membranous EpICD expression together with a positive nodal status as an independent prognostic factor for poor overall survival in the present cohort (Table 3).

### Multi-Tumour Tissue Microarray

Figure 4 shows EpEx expression in different solid malignancies. Colon, ovarian, endometrium, prostate, stomach and non-small cell lung cancer (NSCLC) showed the highest EpEx expression rates in more than 90% of cases. Gallbladder, pancreas, breast and small cell lung cancer (SCLC) showed EpEx expression in 50-90% of cases. In contrast, bladder, esophagus and hepatic cancer showed low expression rates of EpEx (< 50%).

Figure 5 shows the relative expression rates of EpEx+/EpICD-(tr-EpCAM) and EpEx+/EpICD+ (fl-EpCAM) cases in different tumour entities. Endometrium, bladder and stomach showed the highest proportion of EpEx+/EpICD- cases (40-60%). Pancreas, colon, esophagus, ovarian and non-small cell lung cancer (NSCLC) showed a proportion of EpEx+/EpICD- in 20-40% of cases. Small cell lung cancer (SCLC), gallbladder, breast, prostate and hepatic cancer show an EpEx+/EpICD- expression rate varying from 0 to 20%.

Figure 6 shows the proportion of tr-EpCAM in different stages of pre-cancerous lesions compared to colon cancer. The proportion of tr-EpCAM increases from < 10% of cases with low grade dysplastic adenomas to 18% in moderate and high grade dysplasias to 34% in colon carcinoma, respectively. The grade of dysplasia is directly correlated to prognosis of the disease. For this reason, the proportion of tr-EpCAM can be used as a prognostic marker in patients with colon adenomas or carcinomas.

### Discussion

EpCAM is one of the most widely investigated tumour associated antigens in human malignancies. However, the prognostic value of EpCAM expression is still a matter of debate. It was observed that EpCAM overexpression is associated with poor clinical outcome in some epithelial cancers, while additional reports demonstrated EpCAM expression to good prognosis. In the past, it was demonstrated that EpCAM overexpression is detectable in the majority of pancreatic carcinomas and therefore may represents an attractive target for immune-based therapeutic interventions in these tumours.

In the present example the membranous expression pattern of EpCAM in pancreatic cancer was investigated for the first time using two different antibodies directed against EpEX and EpICD domains of the EpCAM antigen. In non-neoplastic pancreatic tissue a high concordance between EpEX and EpICD was observed. In contrast, loss of membranous EpICD expression could be detected in 27% of cancer specimens which was significantly associated with decreased overall survival and correlated with a shortened progression-free interval. It can be concluded that two different EpCAM variants may occur during epithelial carcinogenesis, the inactivated EpEX⁺/EpICD⁺ phenotype representing the full-length protein and an activated remnant transmembrane peptide (EpEX⁺/EpICD⁻) characterized by its cleaved cytoplasmic domain. These two EpCAM variants may have not only prognostic but also therapeutic consequences.

Regulated intramembrane proteolysis activates EpCAM as a mitogenic signal transducer through nuclear translocation of its intracellular domain EpICD. The oncogenic potential of both proteins, full-length EpCAM but also cleaved EpICD on its own was demonstrated by using stable transfected colon carcinoma cells in vivo. In addition, EpICD was found as nuclear complexes in colon carcinoma but not in normal tissue. Moreover, a focal loss of membrane EpEX was described at the invasive margin in colorectal cancer. A reciprocal loss of membrane EpEX but increased nuclear and cytoplasmic accumulation of EpICD was reported in a variety of epithelial carcinomas. Interestingly, compared to the remaining epithelial tumours, in pancreatic cancer a reduced nuclear and cytoplasmic staining pattern for EpICD was observed.

However, despite the interesting observation that reduced or absent membranous expression of either EpEX or EpICD is a frequent event in epithelial carcinomas, none of the prior art studies directly compared the expression pattern of the full-length protein EpCAM with possible truncated variants within the same tumour. Only recently it was shown that high EpCAM-expression was associated with good prognosis in resectable pancreatic cancer by suppressing cell activity. Hence, it was interesting to assess the EpICD status in the EpCAM positive group within this cohort.

As current strategies for EpCAM-based therapies are focusing on monoclonal antibodies against EpEX further studies are warranted to define not only the level of EpCAM target expression within the tumour but also the impact of truncated EpCAM on treatment outcome. One reason for the so far moderate success of EpCAM targeted therapies might be the fact that shedding of EpEX to a soluble ligand seems to be a precondition for subsequent intramembrane cleavage and release of EpICD to the nucleus. The findings according to the present invention that none of the tumours in the present study displayed an EpEX⁻ /EpICD⁺ phenotype may support this assumption. Transactivation followed by ectodomain shedding of the epidermal growth factor receptor (EGFR) family through regulated intramembrane proteolysis generating intracellular signals has received significant attention in understanding the complex signaling networks within cells. In addition, a dual function of the cell adhesion molecule E-Cadherin has been described recently by cleavage of the major protein and the release of an intracellular fragment. As a consequence, cell proliferation and migration are stimulated whereas cell-cell adhesion is inhibited.

Beside of ectodomain shedding by different sheddases, receptor cross talk and remnant peptide signaling within the cell membrane through other transmembrane proteins seems to be crucial steps during carcinogenesis. Importantly, novel insights into the accessibility of EpCAM on normal epithelia vs tumour cells come from recent studies identifying a number of other proteins found in close association with EpCAM within the cell membrane. In pancreatic cancer a complex formation of transmembrane proteins including EpCAM, claudin-7, CD44 and tetraspanin was reported to promote tumour progression rather than the individual molecules on their own. In addition, claudin-7 recruits EpCAM into tetraspanin-enriched membrane microdomains which were shown to be associated with poor prognosis and a reduced disease-free survival in colon cancer. Consequently, efficient cleavage of EpCAM may also depend on interactions with other transmembrane proteins to successfully execute its surface to nucleus missile. Hence, processed for shedding but still membrane anchored remnant EpEX may be recognized by EpCAM specific antibodies but subsequent antitumour effector mechanisms might be insufficient. It is tempting to speculate that patients with tumours with an EpEx⁺/EpICD⁺ phenotype would be better candidates for treatment with EpCAM-specific antibodies compared to EpEx⁺/EpICD⁻ expressing tumours. Another reason for the presence of a truncated form of membranous EpCAM might be mutations of the EpCAM gene. In fact, deletions of the EpCAM gene have been recently described in colorectal cancer patients with Lynch syndrome. Moreover, mutations have been described in a non-malignant disease termed congenital tufting enteropathy.

Taken together, it is shown for the first time with the present example that loss of membranous EpICD expression occurs in a significant subset of pancreatic cancer patients suggesting the presence of two distinct EpCAM variants. Carcinomas representing the truncated transmembrane peptide may be associated with transactivation mediated by ectodomain shedding resulting in a more aggressive phenotype. The absence of the cytoplasmic domain EpICD therefore constitutes a highly relevant biomarker specifically for pancreatic and colorectal cancer patients, especially for those patients which undergo anti-EpCAM targeted therapies, i.e. particularly for patients being treated with EpCAM-specific antibodies (i.e. Catumaxomab, edrecolomab or others).

**Table 1: patient characteristics**

| Variable | | Pancreatic cancer | | |
|---|---|---|---|---|
| | | (n) | (%) | *P* -value (log-rank) |
| Sex (n=89) | | | | |
| | Male | 54 | (61) | 0.48 |
| | Female | 35 | (39) | |
| | | | | |

| Age (n=89) | | | | |
|---|---|---|---|---|
| | Mean | 66 | | |
| | Median | 68 | | |
| | Range | 37-86 | | |

| T stage (n=84) | | | | |
|---|---|---|---|---|
| | pT1 | 7 | (8) | <0.01 |
| | pT2 | 22 | (27) | |
| | pT3 | 48 | (57) | |
| | pT4 | 7 | (8) | |

| Lymph node status (n=84) | | | | |
|---|---|---|---|---|
| | Negative | 35 | (42) | <0.01 |
| | Positive | 49 | (58) | |

| Vessel invasion (n=27) | | | | |
|---|---|---|---|---|
| No | | 13 | (48) | 0.03 |
| Yes | | 14 | (52) | |

| Distant metastasis (n=52) | | | | |
|---|---|---|---|---|
| | No | 47 | (90) | <0.01 |
| | Yes | 5 | (10) | |

| Surgical margin (n=78) | | | | |
|---|---|---|---|---|
| | Negative | 55 | (71) | 0.08 |
| | Positive | 23 | (29) | |

| Stage (UICC) (n=85) | | | | |
|---|---|---|---|---|
| | IA | 7 | (8) | <0.01 |
| | IB | 13 | (15) | |
| | IIA | 14 | (17) | |
| | IIB | 40 | (47) | |
| | III | 6 | (7) | |
| | IV | 5 | (6) | |

| Differentiation (n=89) | | | | |
|---|---|---|---|---|
| | Well | 15 | (17) | <0.01 |
| | Moderate | 45 | (51) | |
| | Poor | 29 | (33) | |

| EpICD expression (n=85) | | | | |
|---|---|---|---|---|
| Positive | | 45 | (53) | 0.01 |
| Negative | | 40 | (47) | |

**Table 2: Correlation of EpICD expression with conventional clinicopathological parameters in EpEX positive tumours**

| | | EpICD expression | | | | | |
|---|---|---|---|---|---|---|---|
| | | Total Patients (n) | Negative | Positive | | | |
| | | | n | % | n | % | *P* |
| Sex (n=68) | | | | | | | |
| | Male | 42 | 14 | (33) | 28 | (67) | 0.91 |
| | Female | 26 | 9 | (35) | 17 | (65) | |

| Age at diagnosis (n=68) | | | | | | | |
|---|---|---|---|---|---|---|---|
| | <65 years | 26 | 11 | (42) | 15 | (58) | 0.24 |
| | ≥65 years | 42 | 12 | (29) | 30 | (71) | |

| Differentiation (n=68) | | | | | | | |
|---|---|---|---|---|---|---|---|
| | G1, 2 | 48 | 15 | (31) | 33 | (69) | 0.48 |
| | G3 | 20 | 8 | (40) | 12 | (60) | |

| T stage (n=65) | | | | | | | |
|---|---|---|---|---|---|---|---|
| | pT1, 2 | 23 | 5 | (22) | 18 | (78) | 0.12 |
| | pT3,4 | 42 | 17 | (40) | 25 | (60) | |

| Lymph node status (n=65) | | | | | | | |
|---|---|---|---|---|---|---|---|
| | No | 26 | 9 | (35) | 17 | (65) | 0.91 |
| | Yes | 39 | 13 | (33) | 26 | (67) | |

| Vessel invasion (n=21) | | | | | | | |
|---|---|---|---|---|---|---|---|
| | No | 12 | 2 | (17) | 10 | (83) | 0.06 |
| | Yes | 9 | 5 | (56) | 4 | (44) | |

| Surgical margin (n=59) | | | | | | | |
|---|---|---|---|---|---|---|---|
| | Negative | 41 | 11 | (27) | 30 | (73) | 0.18 |
| | Positive | 18 | 8 | (44) | 10 | (56) | |

| Stage (UICC) (n=66) | | | | | | | |
|---|---|---|---|---|---|---|---|
| | I | 18 | 4 | (22) | 14 | (78) | 0.18 |
| | II, III, IV | 48 | 19 | (40) | 29 | (60) | |

**Table 3: Multivariate analyses (Cox regression) of various prognostic parameters in the subgroup of patients with EpEX positive tumours**

| | Overall survival | | |
|---|---|---|---|
| | P | RR | 95% CI |
| T stage | 0.26 | 1.3 | 0.7-2.4 |
| Nodal status | 0.03 | 2.4 | 1.0-5.6 |
| Surgical margin | 0.97 | 0.9 | 0.4-2.1 |
| Tumour grade | 0.11 | 1.6 | 0.8-2.9 |
| EpICD expression | 0.02 | 2.3 | 1.1-4.9 |
| RR, relative risk. CI, Confidence Interval. | | | |

## Claims

1. Method for in vitro diagnosing a colon carcinoma (CC) tissue specimen or a pancreas cancer (PC) tissue specimen wherein the specimen is analysed for the presence or absence of the ectodomain of EpCAM (EpEX) as well as for the presence or absence of the intracellular domain of EpCAM (EpICD).

2. Method according to claim 1 wherein specimen is a tissue section, especially a paraffin-embedded tissue section or a fresh frozen section; or a cytocentrifugate from pleura or ascites fluids.

3. Method according to claim 1 or claim 2 wherein the presence of EpEX and the absence of EpICD in the specimen is indicative of a shortened disease-free survival of a patient from which the specimen had been obtained.

4. Method according to any one of claims 1 to 3, wherein the presence or absence of EpEX or the presence or absence of EpICD is analysed by use of an antibody binding specifically to EpEX and/or EpICD, respectively.

5. Method according to any one of claims 1 to 4, wherein the presence or absence of EpEX and the presence or absence of EpICD is analysed by use of antibodies binding specifically to EpEX and EpICD, respectively, and wherein the antibodies are preferably monoclonal antibodies.

6. Method according to claim 4 or 5, wherein the antibodies are labelled.

7. Method according to any one of claims 4 to 6, wherein the antibody specifically binding to EpICD and the antibody specifically binding to EpEX are labelled with different labels, preferably with different fluorescence labels.

8. Method according to any one of claims 4 to 7, wherein the binding of the antibodies to EpEX and/or EpICD is detected by using secondary antibodies binding to the antibodies binding to EpEX and/or EpICD, said secondary antibodies being preferably labelled, especially with a fluorescence label, a luminescence label, a colourigenic label; a radioactive label, a biotin label, or combinations thereof.

9. Method according to any one of claims 1 to 8, wherein the method is an enzyme linked immunosorbent assay (ELISA), an immunocytochemistry assay, a detection of circulating tumour cells or tumour stem cells assay, or a detection of circulating cells or stem cells assay.

10. Method according to any one of claims 1 to 9, wherein the method comprises mass spectroscopy.

11. Kit for detecting EpEX and EpICD in an epithelial cancer tissue specimen in a method according to any one of claims 1 to 10, comprising:
- an epithelial cancer tissue specimen,
- an antibody specifically binding to EpICD; and
- an antibody specifically binding to EpEX.

12. Kit according to claim 11, further comprising detection agents for detecting the antibodies and/or detection means for detection of a binding event between the antibodies to EpICD and/or EpEX.

13. Kit according to claim 11 or 12, wherein the antibodies are monoclonal antibodies.

14. Kit according to any one of claims 11 to 13, wherein it further comprises EpICD and EpEX standards, preferably recombinant EpCAM protein, a recombinant EpICD protein, a recombinant EpEx protein, or combinations thereof.

15. Kit according to any one of claims 11 to 14, wherein the kit additionally contains positive and negative control tissue specimens.
